# EUROPEAN PATENT SPECIFICATION

(11) **EP 4 637 613 B1**
(45) Date of publication and mention of the grant of the patent: **09.09.2026**
(21) Application number: 23837358.3
(22) Date of filing: 21.12.2023
(51) Int. Cl.: A61B 34/30, A61B 90/11, A61B 34/20, A61B 34/10

(54) **SYSTEM FOR A MANIPULATION OF A SURGICAL TOOL BY A USER ACCORDING TO A PLANNED TRAJECTORY**
SYSTEM ZUR MANIPULATION EINES CHIRURGISCHEN WERKZEUGS DURCH EINEN BENUTZER GEMÄSS EINER GEPLANTEN TRAJEKTORIE
SYSTÈME DE MANIPULATION D'UN OUTIL CHIRURGICAL PAR UN UTILISATEUR SELON UNE TRAJECTOIRE PLANIFIÉE

(30) Priority: 23.12.2022 EP 22307030
(43) Date of publication of application: 29.10.2025
(73) Proprietor: Ecential Robotics, 38610 Gieres (FR)
(72) Inventor: VIDAL, Clément, 38610 Gieres (FR); SIEFFERT, Jérôme, 38610 Gieres (FR)
(74) Representative: INNOV-GROUP
(86) International application number: PCT/EP2023/087495
(87) International publication number: WO 2024/133824

(56) References cited:
- EP-A1- 3 821 843
- US-A1- 2022 134 558
- US-A1- 2022 218 422
- US-B2- 10 327 849
- JIANBIN HUANG ET AL: "Development of Adaptive Force-Following Impedance Control for Interactive Robot", 16 June 2018, SAT 2015 18TH INTERNATIONAL CONFERENCE, AUSTIN, TX, USA, SEPTEMBER 24-27, 2015; [LECTURE NOTES IN COMPUTER SCIENCE; LECT.NOTES COMPUTER], SPRINGER, BERLIN, HEIDELBERG, PAGE(S) 15 - 24, ISBN: 978-3-540-74549-5, XP047475619

## Description

### FIELD OF THE INVENTION

The invention relates to a system for a manipulation of a surgical tool, by a user, using a surgical robotic system, to treat an anatomical structure according to a planned trajectory. This system is able to reduce the mechanical load applied to the anatomical structure during a surgical intervention.

### BACKGROUND OF THE INVENTION

Surgical robotic systems are frequently used during surgical interventions to assist a surgeon. The surgical robotic system may guide the positioning and orienting of a surgical tool for the surgeon.

The surgical robotic system comprises a robotic arm having an end effector with a tool holder to accommodate a surgical tool. The tool holder is to be placed with a given position and orientation relative to a surgical target. The surgeon can couple a surgical tool, directly or through an access tube, to the tool holder so that once the tool holder is placed relative to the surgical target, the surgical robotic system assists the surgeon in the guiding of the tool. Thus, the surgeon may operate the surgical tool with the right position and orientation relative to the surgical target during the surgery on an anatomical structure of the patient, such as a bone.

The surgeon may use the surgical robotic system to drill one or several holes into one or several bones of a patient, and then to implant a screw into each respective drilled hole. The surgical target corresponds to a specific position and orientation of the hole to be drilled on a bone such as a vertebra, or a fractured bone of the patient. For example, in spine surgery, several surgical targets may be located on several vertebrae of the patient.

The tool holder maintains the tool in a specific position and orientation relative to the surgical target to precisely guide the surgeon into drilling the hole and then implanting the screw. Thus, the surgical robotic system assists the surgeon in drilling the hole and then implanting the screw into the drilled hole.

However, it is observed that the anatomical structure is subjected to significant force during the screwing and that the surgical robotic system is often under excessive tension at the end of screwing. When the surgeon inserts the screw, he exerts forces on the robotic arm, part of which is compensated by the surgical robotic system, but there are also variable deviations from the planned axis that cause the actual axis to move a little randomly during screwing. Those variable deviations can come from a slight misalignment of the screw with the tool axis, a slight misalignment of the screw with the drilling axis or other various errors such as uneven bone surface (facet joint, transverse process...) or soft tissue pressure. The actual screwing axis can then be shifted by some 1/10th mm from the planned screwing axis.

Once the screw starts to engage with the bone, a strong mechanical bond is formed between the screw and the bone and therefore with the surgical robotic system. After this, it is very difficult to modify the screwing axis without exercising very important force on the screwdriver. Since the actual screwing axis differs from the planned screwing axis, it can generate increased force applied by the surgical robotic system onto the patient anatomy since the surgical robotic system tries to align the actual screwing axis with the planned screwing axis and fight against the forces created by this strong mechanical bond. This is problematic because it can cause various issues like unscrewing the screw, risk of damaging the bone, create undesirable locking with the tool, etc.

Document US 2022/218422 A1 teaches a system according to the preamble of claim 1.

A purpose of the invention is to propose a system able to reduce the mechanical load applied to the anatomical structure during a surgical intervention.

### BRIEF DESCRIPTION OF THE INVENTION

It is an object of this invention to provide a system for a manipulation of a surgical tool by a user to treat an anatomical structure according to a planned trajectory, said system comprising:
- a surgical robotic system configured to align the surgical tool according to the planned trajectory, the surgical robotic system comprising:
   * a robotic arm comprising a plurality of segments driven by motors, the robotic arm being able to be positioned according to a reference pose configured to align the surgical tool with the planned trajectory;
   * at least one load measurement means to measure a mechanical load applied to the robotic arm;
   * an end-effector mechanically coupled to a distal end of the robotic arm and comprising a tool holder to accommodate the surgical tool;
- a control unit coupled to the surgical robotic system and configured to implement at least one controlled loop to reduce a mechanical load applied to the anatomical structure once the robotic arm is positioned according to the reference pose, the controlled loop comprising:
   a. a step of measuring the mechanical load applied to the robotic arm using the at least one load measurement means, the mechanical load comprising an inherent mechanical load which is inherent to the robotic arm and an external mechanical load which is applied to the motors of the robotic arm by an external influence;
   b. a step of determining an adjusted command for at least one motor, using the following method:
      i. determining an external mechanical load value based on the measured load;
      ii. determining a compensation term based on the external mechanical load value and on data stored in the control unit, said data comprising at least one set of parameters defining a respective compensation model generated by the control unit, each compensation model defining, for each external mechanical load value, a corresponding compensation term which increases with the external mechanical load value;
      iii. generating the adjusted command based on the compensation term and the reference pose;
   c. a step of applying to said at least one motor the respective adjusted command to reduce the mechanical load applied to the anatomical structure.

In the present text, the term "load" is to be understood as meaning the mechanical load applied to the robotic arm, to a component of the robotic arm, and/or to the anatomical structure. The invention does not include method claims.

According to other advantageous and non-limiting features of the present disclosure, taken alone or in any technically feasible combination:
- the controlled loop comprises, before step ii), a step of storing and/or updating, in the data stored in the control unit, a robotic arm pose information with a current robotic arm pose, this step being done only once during a first iteration of the controlled loop or repeated for each iteration of the controlled loop.
- the at least one load measurement means comprises a plurality of torque measurement sensors configured to measure a torque of each motor and the control unit is configured to determine the external mechanical load based on the external torque value associated to each motor by subtracting from the measured torque value of each motor inherent torque values of the robotic arm;
- the at least one load measurement means comprises a force-torque sensor configured to measure a wrench applied to the robotic arm and the control unit is configured to determine the external mechanical load based on the external wrench value by subtracting from the measured wrench value inherent wrench values of the robotic arm;
- The system comprises a localization system coupled to the control unit;
- The system comprises a patient tracker, localizable by the localization system, rigidly attached to a base structure fixed with respect to the anatomical structure and wherein the surgical robotic system is servo-controlled on the movements of the patient tracker;
- The control loop is configured to update the reference pose of the robotic arm at the beginning of each iteration of the controlled loop to take into account the servoing of the surgical robotic system on the movements of the patient tracker;
- the system comprises a robot tracker, localizable by the localization system, rigidly attached to a part of the surgical robotic system;
- the system comprises an alarm system to inform a user when at least one safety criterion of the robotic arm is not met, the safety criterion being chosen among:
   - a maximal allowed angular difference between a current axis of the surgical tool and an axis of the planned trajectory; and
   - a maximal allowed distance difference between an estimated end point position of the tool tip and a planned end point position of the planned trajectory;
   wherein the control unit is configured to:
   - determine the current axis of the surgical tool and the estimated end point position of the tool tip along the current axis of the surgical tool;
   - compute an angular difference between the current axis of the surgical tool and the axis of the planned trajectory;
   - compute a distance difference between the estimated end point position of the tool tip and the planned end point position of the planned trajectory;
   - if the angular difference and/or the distance difference is greater than the respective safety criterion, send a signal to trigger the alarm system to alert the user.
- the stored data comprise at least two sets of parameters defining each a different compensation model and the control unit is configured to select a compensation model among said different compensation models and to determine, for each motor, the compensation term of step b) using the selected compensation model and the external mechanical load value.
- The system comprises a button configured to, when pressed by a user, change the compensation model used by the control unit in step b).
- the system comprises a tool tracker, localizable by the localization system, rigidly attached to the surgical tool and the control unit is configured to implement, before step b), a step a') of automatically changing the compensation model used in step b) according to a current position of a tool tip of the surgical tool along the planned trajectory.
- the control unit is configured to implement the step a') using the following method:
   - determining the current position of the tool tip of the surgical tool along the planned trajectory using a position information of the tool tracker from the localization system;
   - computing a distance between the current position of the tool tip with a planned end point position of the planned trajectory;
   - if the distance is lower than a proximity value, changing the compensation model used in step b).
- the stored data comprise a maximal allowed force value applicable to the anatomical structure and the control unit is configured to :
   - based on the maximal allowed force value, compute a maximal external torque value for each motor or a maximal external wrench value for the robotic arm ;
   - send a stop command to the robotic arm, to stop movement of the robotic arm if the extemal torque value applied to a motor or the external wrench value measured in step b), is greater than the respective maximal external torque value or maximal allowed external wrench value.

### BRIEF DESCRIPTION OF THE DRAWINGS

Further features and advantages of the invention will be apparent from the following description, based on the appended drawings wherein:
- FIG. 1 illustrates a surgical scene including the surgical robotic system according to the invention;
- FIG. 2 illustrates a surgical robotic system according to the invention, a patient tracker and a robot tracker;
- FIG. 3 illustrates two different compensation models according to the invention;
- FIG. 4 is a flow chart representing a first embodiment of the method of reducing the mechanical load applied to the anatomical structure as the user manipulates the surgical tool;
- FIG. 5 is a flow chart representing a second embodiment of the method of reducing the mechanical load applied to the anatomical structure as the user manipulates the surgical tool;
- FIG. 6 is a flow chart representing a third embodiment of the method of reducing the mechanical load applied to the anatomical structure as the user manipulates the surgical tool;
- FIG. 7 is a flow chart representing a fourth embodiment of the method of reducing the mechanical load applied to the anatomical structure as the user manipulates the surgical tool.

For sake of legibility of the drawings, the figures are not necessarily drawn to scale.

The reference signs identical from one figure to another one designate the same elements or elements fulfilling the same function.

### DETAILED DESCRIPTION OF EMBODIMENTS

The invention uses a surgical robotic system that holds a surgical tool and may use a localization system such as presented below.

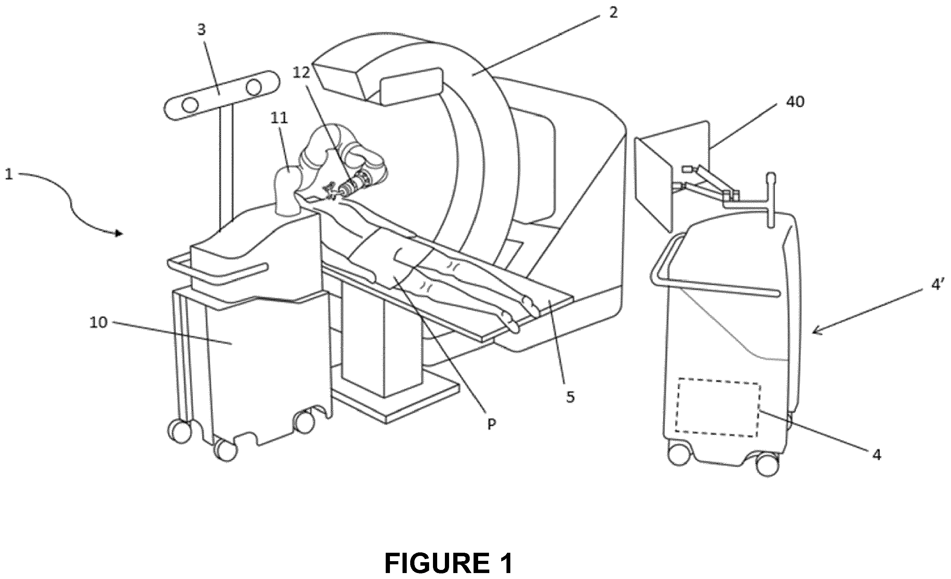

FIG. 1 illustrates a surgical scene comprising said systems in order to carry out a surgical intervention to treat an anatomical structure.

A patient P lies on an operating table 5.

The surgical robotic system 1 comprises a base 10 and a robotic arm 11 carrying a surgical tool and is placed in the vicinity of the operating table 5.

The invention may comprise an X-ray imaging system 2 also placed in the vicinity of the operating table, to acquire 2D X-ray images of the anatomical structure to be treated. This X-ray imaging system is not required to operate the system according to the invention.

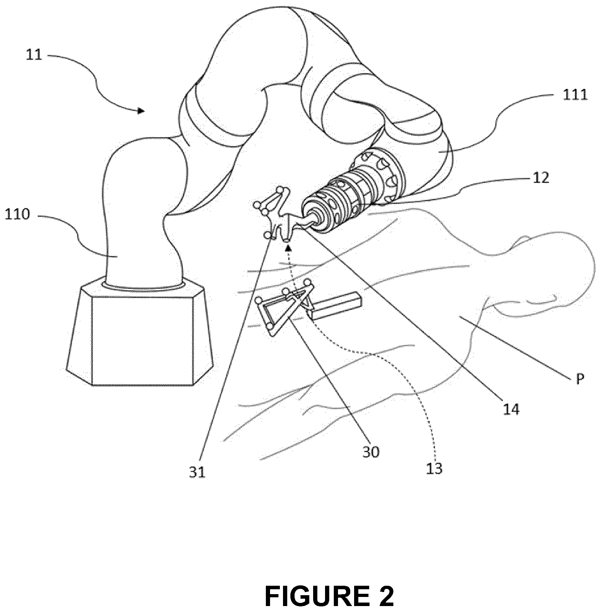

As shown in FIG. 2, the patient and the surgical system may comprise at least one respective tracker rigidly attached thereto, for example via a mechanical or magnetic attachment, said trackers being localized by a localization system 3.

A control unit 4 is coupled to the surgical robotic system (in particular, to a controller of the surgical robotic system) and to the X-ray imaging system if available. The control unit 4 comprises at least one processor configured to implement algorithms designed to carry out the method that will be described below. In particular, the control unit may be configured to perform the following steps: receive the 3D image (whether acquired by the X-ray imaging system 2 or by another system), receive a planning done on the 3D image or allow a user planning the surgical intervention on the 3D image to obtain a planned trajectory of the surgical tool to treat the anatomical structure, determine a reference pose of the robotic arm in which the robotic arm is able to align the surgical tool with its planned trajectory, compute the command to move the robotic arm to position it according to the reference pose, compute relative position of the trackers based on data received from the localization system, control the surgical robotic system to maintain the reference pose taking into account the computed relative positions of the trackers.

The control unit may also be coupled to a user interface 40. The user interface may include one or more screens.

### X-ray imaging system

The X-ray imaging system comprises at least one X-ray source and at least one X-ray image detector. The X-ray imaging system produces at least one 2D X-ray image that is the result of a conical projection of a patient anatomy, wherein the tip of the cone is approximately the central point of the X-ray source and the basis of the cone is approximately the portion of the X-ray image detector that is reached by X-ray beams that have been collimated in a given shape and orientation.

For example, the X-ray imaging system can be a conventional C-arm, or any Cone-Beam Computed Tomography (CBCT), such as the Surgivisio device (Surgivisio, Gieres, France), or Vision FD Vario 3D (Ziehm), CIOS Spin Mobile 3D (Siemens), Airo (Stryker), Loop-X (Brainlab), O-arm (Medtronic).

A conventional C-arm is designed to allow the X-ray source and X-ray detector to rotate along a C-shaped gantry while obtaining projection images of the patient placed between the X-ray source and the X-ray detector of the gantry.

A CBCT has a mobile X-ray source and a mobile X-ray image detector, wherein the X-ray source and the X-ray image detector have motorized motions, moving together or independently. A CBCT can have a C-arm shape or an O-arm shape. It can be used to acquire a set of 2D X-ray images over approximately 180° of orbital rotation that can be combined with translations and from which a 3D image can be reconstructed using tomography algorithms or tomosynthesis algorithms.

The X-ray imaging system may be motorized, notably the C-shaped arm may comprise motors allowing movement horizontally, vertically and around the swivel axes, so that 2D X-ray images of the patient are produced from almost any angle. Each motor is associated to an encoder that provides at any time the relative position of the medical imaging system with respect to a reference position. When a 2D X-ray image is acquired, the corresponding position of the imaging system is recorded. Thus, each 2D image is recorded in the referential of the imaging system.

In some embodiments, a 3D image of the patient may be obtained during the surgery, using the X-ray imaging system itself if it is a CBCT. Said 3D image is registered with respect to a tracker attached to the anatomical structure using known methods of calibration and navigation.

In other embodiments, the 3D image may be acquired prior to surgery using a Computed Tomography (CT) device or another CBCT device. Said 3D image of the patient is registered with respect to the tracker attached to the anatomical structure using a 3D registration method that can use many techniques, such as (i) a collection of surface points using the localizing system and their fitting on the anatomical structure like in the technique provided by 7D Surgical (North York, Canada), or (ii) the acquisition of 2D X-ray images calibrated with respect to the tracker attached to the anatomical structure and used for registration with the 3D image like in the technique provided in the Mazor X robotic system (Medtronic), or (iii) any registration technique using localized ultrasound images, fiducials, anatomical points and the like.

### Surgical robotic system

A surgical robotic system 1, in the sense of the present invention, may comprise, with reference to FIG.1 and 2:
- a base 10, which may be a movable cart (as shown in FIG. 1) or may be attached to the operating table (this embodiment has not been illustrated),
- a robotic arm 11 comprising a plurality of segments driven by motors, the robotic arm 11 having a proximal end 110 extending from the base, and a distal end 111 opposite to the proximal end,
- at least one load measurement means to measure the load applied to the robotic arm 11;
- an end-effector 12 mechanically coupled to the distal end 111 of the robotic arm and comprising a tool holder 14 to accommodate a surgical tool;
- a controller configured to controllably move the robotic arm according to a planned trajectory.

The robotic arm 11 comprises a plurality of degrees of freedom in translation and/or rotation and can be positioned according to a great variety of pose. Usually, the robotic arm comprises at least five, and preferably six or seven motorized degrees of freedom. To that end, the robotic arm comprises a plurality of articulated segments driven by motors. A robotic arm can be for example the LBR Med^{™} robot provided by KUKA (Germany). Said robotic arm can be controlled in an autonomous mode according to desired targets and trajectories, or it can be manipulated using a collaborative mode (cobot), or it can be telemanipulated using a master control device, and a combination of these different modes can be used on the same surgical robotic system.

The surgical robotic system can be active, in the sense that it holds and moves a powered surgical tool that interacts directly with the anatomical structure, or passive in the sense that it holds a guide in a predefined position with respect to the anatomical structure into which a surgical tool is inserted by a user. For example, a powered drill is mounted on the robotic arm tool holder and actively drills the bone along a predefined path until an end point of a selected linear trajectory is reached. Or a screwdriver can be inserted in the tool holder and manually be rotated by the surgeon to screw a screw in a previously drilled hole in the bone along a predefined path until an end point of a selected linear trajectory is reached.

The tool holder 14 represented on the figures comprises a tubular shape to accommodate the surgical tool but other shape could be used, for example in the case of a powered tool, the tool holder could be a simple interface to fix the tool to the end-effector.

The surgical tool is not illustrated in FIG. 2 but its position is represented schematically by reference 13.

Said surgical tool can be a powered drill, a powered saw, a powered burr or mill, an ultrasonic milling device, a radiofrequency or microwave or cryogenics ablation needle or a screwdriver, or any device that can interact with the anatomical structure to be treated. For example, a powered burr can be used to remove a volume of bone where a tumor has been detected, the surgical robotic system being controlled to have the burr tip execute a 3D complex path trajectory corresponding to the bone volume to be removed. The surgical tool can move relative to the tool holder in at least one degree of freedom, for example in two degrees of freedom: in translation along the tool axis and in rotation around the tool axis.

The surgical robotic system 1 further comprises at least one load measurement means (not visible on the figures) to measure the load applied to the robotic arm, the measured load being a torque or a wrench. The torque is a measured load in the joint space and comprises one value for each motor (could also be expressed as a vector with a component for each motor). The wrench is a measured load in the cartesian space and comprises one vector for the robotic arm, this vector comprising six components (3 translations and 3 rotations). This measured load is used by the control unit to determine the external load value applied to the robotic arm or, in other words, the external torque applied to each motor or the external wrench applied to the robotic arm. The external load is an estimation of the load applied to the robotic arm which is not inherent to the robotic arm. In other words, the external load is an estimation of the load applied to the motor by an external influence, for example here by a surgeon.

In a first embodiment, said at least one load measurement means comprises a plurality of torque measurement sensors such as electrical current sensors or deformation measurement sensors, configured to measure the torque of each motor. Advantageously, each motor has its own associated torque measurement sensor. Those torques measurement sensors are generally attached between each of the motors, or gearbox assembly, and the segment body of the robotic arm. The external torque value of each motor is then determined by deducting from the measured torque value of each motor the inherent torques values of the robotic arm, using a dynamic model, such as: the torque produced to counter gravity, the torque consumed to counter the effects of friction or viscoelasticity of the motor itself, the torque needed to accelerate or brake the robotic arm.

Optionally, the control unit is then configured to determine the external wrench value W using the external torque value C and the inverse of the Jacobian transpose Jt associated to the robotic arm pose using the following formula with C being a vector comprising the external torque of each motor, Jt being the Jacobian transpose and W a vector comprising the six components of the external wrench W. $W = {\left(Jt\right)}^{- 1} ∗ C$

In a second embodiment, the at least one load measurement means comprises a force-torque sensor disposed on the surgical robotic system and configured to measure the wrench W applied to the robotic arm. The force-torque sensor can be disposed on the base of the surgical robotic system, on a segment of the robotic arm, on the flange or extremity of the robotic arm or on the tool holder. The external wrench is then determined by deducting from the measured wrench value the inherent wrench value of the robotic arm similarly to the previously described determination of the external torque value from the measured torque value using dynamic model.

Optionally, the control unit is then configured to determine the external torque value associated to each motor using the external wrench value and the Jacobian transpose Jt associated to the robotic arm pose using the following formula. $C = Jt ∗ W$

### Control unit, stored data and compensation model(s)

In the embodiment of FIG. 1, the control unit 4 is embedded in a separate station 4' from the surgical robotic system 1 and is configured to communicate wirelessly or by wires with the robotic arm 11. This separate station 4' here also comprises the user interface 40 and may include batteries.

In other embodiments (not illustrated), the control unit may be embedded in the base 10.

The control unit 4 comprises at least one processor configured to implement algorithms designed to carry out the controlled loop that will be described below. The control unit 4 also comprises at least one data storage device to store data such as the maximal allowed force value Fmax applicable to the targeted anatomical structure, the maximal allowed deviation Dmax at the tool tip, the robotic arm pose information if needed, and at least one set of parameters defining a compensation model. Generally, the maximal allowed force value Fmax is comprised between 25 and 35 N and the maximal allowed deviation Dmax is comprised between 0.1 and 3.0 mm.

The robotic arm pose information may correspond to the current robotic arm pose and be continuously, or periodically, updated while the robotic arm is in use or be stored only once when the surgeon starts to interact with the surgical tool to reduce the burden on the control unit. This last option is advantageous if there is no significant change in the robot arm pose while in use. This robotic arm pose information is mostly used when working with torque value to determine the lever arm of each motor.

Preferably, the control unit 4 stores at least two set of parameters defining each a different compensation model, and generates each of the at least two compensation models using the stored data.

The compensation model defines for each external load L value (either an external torque value or an external wrench value) a compensation term Δ which increases with the external load value L. The compensation term is used to generate, for each motor, an adjusted command allowing a controlled deviation from the planned trajectory to reduce the load applied to the anatomical structure in relation with the external load value and according to the stored maximal allowed force and deviation value.

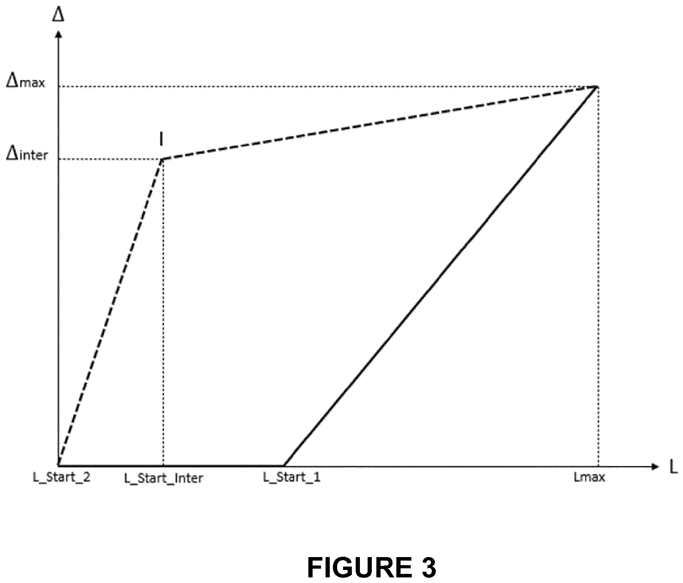

FIG. 3 illustrates two different compensation models according to the invention: a first compensation model (solid line curve on the figure), and a second compensation model (dotted line curve on the figure).

Each compensation model is defined by a maximal compensation term Δmax and a maximal extemal load value Lmax and a set of parameters specific to the compensation model. The control unit computes the maximal compensation term and the maximal external load value, using if needed the robotic arm pose information, and respectively the maximal deviation value and maximal allowed force value.

The set of parameters, specific to each compensation model, comprises at least one load start ratio L_Start_Ratio1. The load start ratio corresponds to a ratio of the maximal external load value Lmax to define the external load start value L_start above which there is a non-zero compensation term. On FIG. 3, L_Start_1 and L_Start_2 are, respectively, the external load start value of the first model and of the second model. $L_start = L_Start_Ratio 1 ∗ Lmax$

If the external load value is lower than the external load start value, no modification will be done to the current command. Of course, the load start ratio L_Start_Ratio1 can be equal to zero, and so the external load start value L_Start, as illustrated by the second compensation model. However, it is preferred that at least one compensation model, here the first compensation model, has a non-zero value for the load start ratio L_Start_ratio1. Indeed, the accuracy of the robotic arm must be preserved as much as possible for low external load value by having for those values a null compensation term to prevent any deviation from the planned trajectory.

If the external load L is greater than its associated maximal external load value Lmax, the control unit 4 is configured to send a stop command to the robotic arm 11 to stop its movement. Indeed, an extemal load value greater than the maximal external load value means that the load applied by the tool on the anatomical structure is not tolerable and poses a risk to the patient or that there is an issue which much be resolved before continuing the operation.

The compensation term Δ is equal to 0 when the external load L is equal or lower than the extemal load start value L_Start, and is equal to the maximal compensation term Δmax when the external load L is equal to the maximal external load value Lmax. Between the external load start value L_Start and the maximal extemal load value Lmax, the compensation term Δ can be determined using a linear function, as represented in the first compensation model of FIG. 3, or using piecewise linear functions to define multiple compensation steps, as represented in the second compensation model which comprised two steps delimited by an intermediary point I.

Here, the second model comprises an intermediary point I defined by two additional parameters: a compensation start ratio Δ_Start_ratio and a second load start ratio L_Start_ratio2. The compensation start ratio Δ_Start_ratio corresponds to a ratio of the maximal compensation term Δmax to defined the intermediary compensation term, Δinter, associated to the intermediary point I. $Δinter = Δ _ Start_Ratio ∗ Δmax$

The second load start ratio L_Start_ratio2 correspond to another ratio of the maximal external load value Lmax to define the intermediary external load start value L_Start_Inter associated to the intermediary point I. $L_Start_Inter = L_Start_Ratio 2 ∗ Lmax$

The compensation term Δ evolves, with the external load value L, differently before and after this intermediary external load start value L_Start_Inter.

Of course, it is possible to use more intermediary points and/or more complex compensation model such as temporal law or binomial law.

As previously mentioned, the external load L value can be an external torque C value or an extemal wrench W value.

In the case of an external torque value, each compensation model is defined in a joint space and so is specific to each motor taking into account the robotic arm pose information and more particularly the lever arm of each motor. In other words, each motor has its own version of the compensation models used. More particularly, as shown in the following equation, each i motor has its own maximal external torque value Lmax_i, and its own maximal compensation term Δmax_i computed by the control unit using the stored maximal allowed force Fmax value, the stored maximal allowed deviation Dmax and the lever arm Li the motor has with respect to the entry point of the planned trajectory. $Lmax_i = \frac{Fmax}{Li} ; Δmax_i = \frac{Dmax}{Li}$

The lever arm Li is the distance between the axis of the i motor and the entry point of the planned trajectory. The lever arm Li is determined using the robotic arm pose information, which comprises the vectors joint positions, and the information about the length of each segment of the robotic arm.

Advantageously, in the case of screwing a screw, a more accurate lever arm is calculated using an estimate of the position of the screw head placed at the entry point instead of using only the entry point. In other word, an offset is added to the entry point to account for the screw.

In the case of an external wrench value, each compensation model is defined in a Cartesian space and those compensation models are declined for each j component of the wrench value (three translations and three rotations). In this case, the maximal wrench value Lmax_j and the maximal compensation term Δmax_j associated to each j component of the wrench value are respectively equal to the associated maximal allowed force value Fmax_j and maximal allowed deviation Dmax_j, Fmax and Dmax being defined here as vectors comprising j components.

Working in the Cartesian space allows for more transparency and more control over the deviation from the planned trajectory by being able to restrict differently the deviation along specific axis and/or rotation axis since Fmax and Dmax are vectors and so it is possible to use different value for Fmax_j and Dmax_j for each j component.

### Localization system

A localization system 3 is used that can localize the three parameters of position and the three parameters of orientation of any tracker mounted rigidly, for example via a mechanical or magnetic attachment, on anatomical structures such as bones, or devices, such as surgical tools or a subsystem of the surgical robotic system. Said localization system 3 can be an optical system (such as Aurora from NDI, Canada), or any combination of optical, electromagnetic, ultrasonic, inertia measurement devices and sensors, or passive electro-mechanical arm with encoders.

A first tracker 30 may be rigidly attached to a base structure that is assumed to be fixed with respect to the anatomical structure to be treated, therefore said first tracker is supposed to be fixed with respect to the anatomical structure. Said base structure can be the anatomical structure itself, or an adjacent structure, or any mechanical fixation fixed to the patient or to the operating table if the motions between said base structure and the anatomical structure to be treated can be neglected for the required accuracy.

A second tracker 31 is rigidly attached to a part of the surgical robotic system itself, such as the base. The kinematic model of the robotic arm is known for any position of the robotic arm using its encoders values and therefore by a simple combination, the axis of the surgical tool is known in a coordinate system attached to the second tracker at any time. This second tracker could also be directly integrated to the surgical robotic system.

Optionally, a third tracker can be used (not visible on the figures), and rigidly attached to the surgical tool to know the position and orientation of the tooltip of the surgical tool.

### Anatomical structure

The anatomical structure to be treated is usually a bone that may be drilled, burred, and/or milled in order to place an implant or to free some space for any clinical reason.

The method may be applied successively to several parts of one bone or to several bones. For example, the method may be used for placing screws in both pedicles of several vertebrae.

### Operation of the surgical robotic system

The surgical robotic system may be operated as follows.

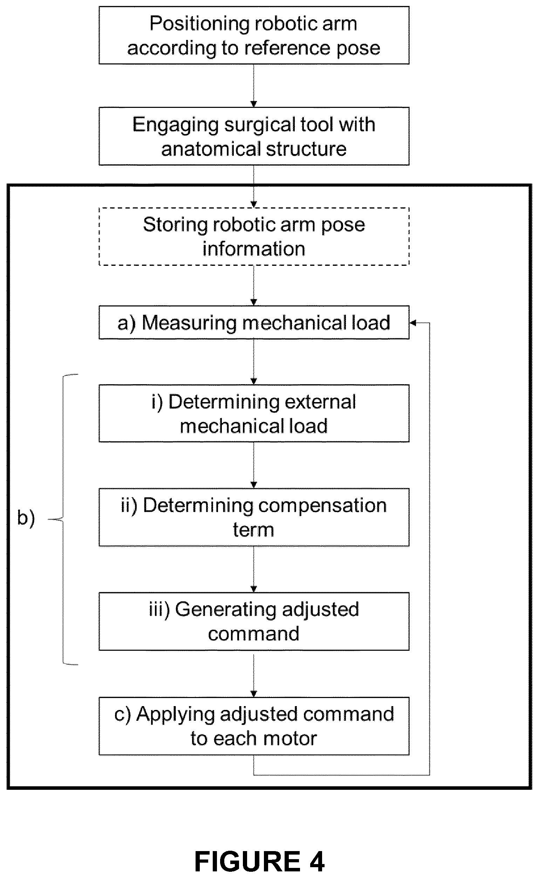

FIGS. 4 to 7 present flow charts of various embodiments of the method of operation of the surgical system. In these flow charts, only the main steps have been represented. These flow charts are not to be intended to be limitative and may be combined, if appropriate. In addition, steps that do not depend from each other may be interchanged. Steps surrounded by a dotted frame are optional. The steps enclosed in the thick solid line frame are carried out by the control unit.

At the beginning of surgery, the patient is equipped with a first tracker 30 (called "patient tracker") detectable by the localization system 3.

A 3D image is acquired either at the beginning of surgery using the X-ray imaging system itself (CBCT) or prior to surgery with another imaging system (CT or CBCT), and as described above said 3D image is registered with respect to the patient tracker 30.

The trajectory of the surgical tool is planned in the 3D image, generally this trajectory consists of a movement along a planned axis to reach a predetermined end point. For that purpose, a surgical planning software is used to define the trajectory interactively or automatically in the 3D image. Optionally, a plurality of trajectories is planned, for example if the user needs to drill several holes, and the method is repeated for each trajectory. A reference pose for the robotic arm 11 is also determined using the planned trajectory. This reference pose corresponds to a pose of the robotic arm 11 in which, when equipped with the surgical tool 13, the surgical tool 13 is aligned with the planned trajectory in the absence of external load.

The surgical robotic system 1, which may be mobile on the wheels of the cart forming the base of the robotic arm, is brought near the surgical table.

As shown on FIG. 2, a second tracker 31 (called "robot tracker") is mounted on the robotic arm 11, on the surgical tool directly or on any sub-system of the surgical robotic system. The calibration of the robot tracker with respect to the surgical tool can use several known calibration methods.

A third tracker (called "tool tracker") can be mounted on the surgical tool 13 before or after the tool being assembled to the surgical robotic system.

In a preferred embodiment, the position of the tool tracker on the surgical tool is reproduced and always the same, and a localized pointer is used to check that a particular point of the surgical tool has precisely the expected coordinates with respect to the tool tracker.

In another preferred embodiment, the localized pointer is used to digitize at least three precisely defined points on the surgical tool and a point-based calibration is applied.

The surgical robotic system is then moved manually (cobot) or automatically to be positioned according to the reference pose, to align the surgical tool to the planned trajectory, until it reaches an entry point on the bone surface. The surgical robotic system can then be servo-controlled on the movements of the patient tracker, using the robot tracker and/or the tool tracker, in order to maintain alignment with the entry point position on the bone, compensating for any motions of the bone due to patient's breathing or any mechanical interactions. This servo-control is generally done by adjusting the pose of the robotic arm in order to maintain a constant relationship between the position of the patient tracker and the position of the robot and/or tool tracker. In other words, the movement of the robot tracker follows the movement of the patient tracker to keep a constant distance between both trackers in the absence of external interaction from the user.

Then, the surgical robotic system 1 is equipped with a surgical tool 13 and the user can start manipulating the surgical tool inside the bone along its planned trajectory by manually translating and/or rotating the tool in relation with the tool holder.

The control unit 4 is then configured to implement at least one controlled loop to reduce a load applied to the anatomical structure once the robotic arm is positioned according to the reference pose. Advantageously, the first control loop is implemented once the surgical tool engages with the anatomical structure.

As shown in FIGS. 4-7, the control loop comprises:
a) A step of measuring the load applied to the robotic arm using the at least one load measurement means;
b) A step of determining an adjusted command, for each motor, using the following method:
   i. determining the external torque value applied to the motor, or an external wrench value applied to the robotic arm, using the measured load value,
   ii. determining a compensation term based on the external torque value, or the extemal wrench value, and data stored in the control unit;
   iii. generating the adjusted command based on the compensation term and the reference pose,
c) A step of applying to each motor the respective adjusted command to reduce the load applied to the anatomical structure.

More particularly, the control unit determines the compensation term using the previously described compensation model generated using the stored data. If an external torque is used during step ii), the compensation term is directly expressed in the joint space and is added to the reference pose of the robotic arm to generate the adjusted command of each motor. If an external wrench value is used during step ii), an extra step is required since the compensation term is obtained in a cartesian space and so a conversion need to be done to obtain an adjusted command in the joint space. To do that it is possible to either directly convert the compensation term into the joint space using the inverse Jacobian and add this compensation term to the reference pose, or to directly compute an adjusted command in the cartesian space and then to convert it into an adjusted command in the joint space using inverse kinematic.

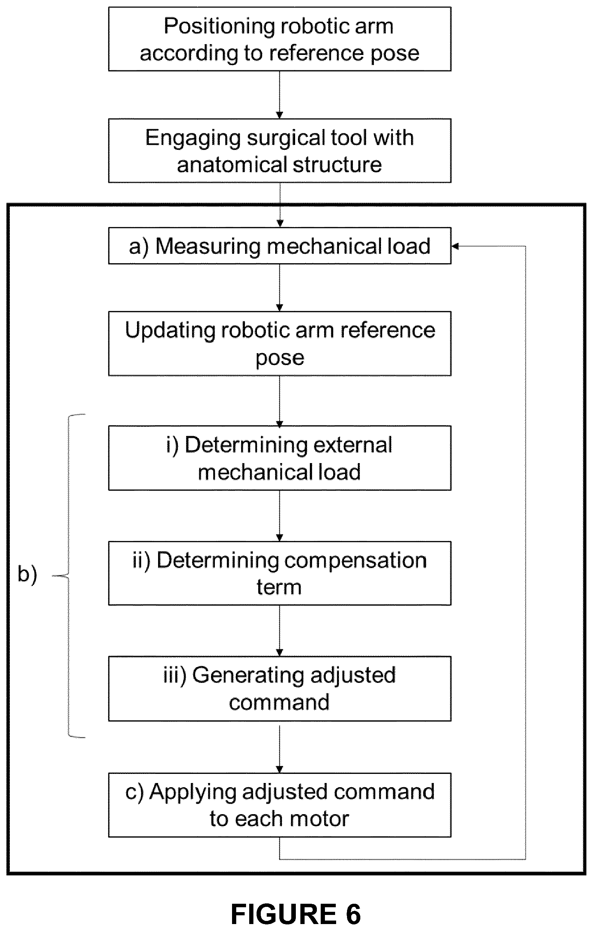

In the case where the surgical robotic system is servo-controlled on the movement of the patient tracker (see FIG.6), the reference pose of the robotic arm is updated at the beginning of each iteration of the controlled loop to take into account this servoing when generating the adjusted command of each motor. As a reminder, the reference pose does not consider the interaction of the user with the robotic arm, through the interaction of the user with the surgical tool, but only the planned trajectory and, if the robotic arm is servo-controlled, the movement of the patient tracker in relation with the robot tracker position.

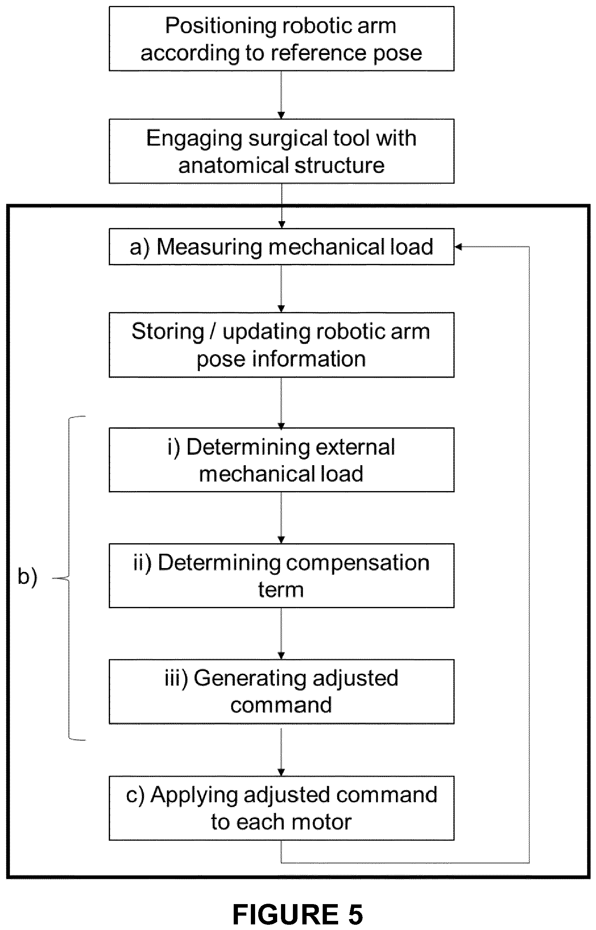

Optionally, the controlled loop may comprise, before step ii) a step of storing and/or updating, in the data stored in the control unit, the robotic arm pose information with the current robotic arm pose. This step can be done only once during a first iteration of the controlled loop (see FIG. 4) or repeated for each iteration of the controlled loop (see FIG. 5). This robotic arm pose information is mostly used when working with torque values to have access to the lever arm information of each motor for the determination of the compensation term of step ii). Contrary to the reference pose, the current robotic arm pose considers the interaction of the user with the robotic arm through the interaction of the user with the surgical tool.

Advantageously, the stored data comprise for each motor, at least two sets of parameters defining each a different compensation model to allow the possibility of changing the compensation model used by the control unit during the operation of the robotic surgical system. The first compensation model is used as a default model and the second model is manually or automatically selected by the control unit 4 during the operation of the surgical robotic system 1 to change the compensation model used to determine the compensation term in step b). The second model differs from the first model in that it allows, for a given extemal torque value or extemal wrench value, a greater compensation term to reduce more the load applied to the anatomical structure by allowing more deviation from the planned trajectory.

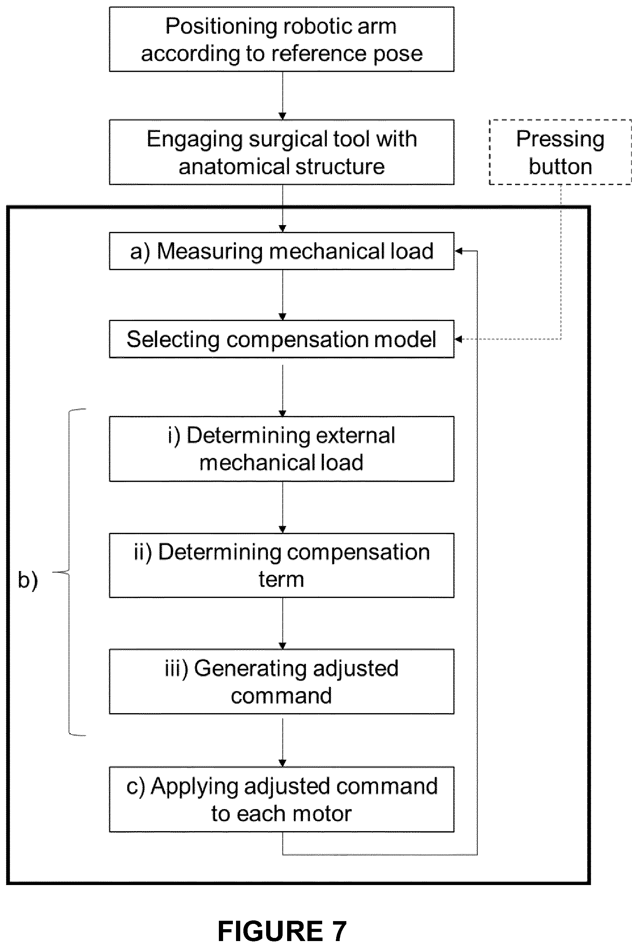

In a first embodiment, the change of compensation model is done manually by the user by pressing a button (see FIG. 7). This button can be placed on the tool, on the separate station 4' embedding the control unit 4 or any part of the surgical robotic system 1. When this button is pressed, the control unit 4 is configured to select the second compensation model for each motor and will determine the next compensation terms using this second compensation model. Instead of pressing a button, other types of commands such as voice or gesture commands could also be considered.

In a second embodiment, the change of compensation model is done automatically by the control unit 4. This is possible if there is a way to track the position of the tool tip, such as using the tool tracker, localizable by the localization system 3, rigidly attached to the surgical tool. The control unit 4 is configured to implement, before step b) and/or step a), a step a') of automatically changing the compensation model used in step b) according to the position of the tool tip of the surgical tool 13 along the planned trajectory using the following method:
- determining the current position of the tool tip of the surgical tool 13 along the planned trajectory using the position information of the tool tracker from the localization system 3;
- computing the distance between the current position of the tool tip with the planned end point position of the planned trajectory;
- if the distance is lower than a proximity value, changing the compensation model used in step b).

Optionally, it could also be possible to use three or more compensation models with for example: a first compensation model used by default, a second compensation model that triggers automatically when the proximity criterion is met, and a third compensation model that can be activated manually when there is a need to change the compensation model before the proximity criterion of the second compensation model is met.

This controlled loop is then repeated all along the manipulation of the surgical tool 13 by the user, at various instants, for example each 5 ms.

Advantageously, the system comprises an alarm system to inform the user when at least one safety criterion of the robotic arm is not met, the safety criterion being chosen among:
- a maximal allowed angular difference between a current axis of the surgical tool and an axis of the planned trajectory; and
- a maximal allowed distance difference between an estimated end point position of the tool tip and a planned end point position of the planned trajectory;
and the control unit 4 is configured to:
- determine a current axis of the surgical tool 13 and an estimated end point position of the tool tip along the current axis of the surgical tool 13;
- compute an angular difference between the current axis of the surgical tool 13 and an axis of the planned trajectory,
- compute a distance difference between the estimated end point position of the tool tip and a planned end point position of the planned trajectory;
- if the angular difference and/or the distance difference is greater than the respective safety criterion, send a signal to trigger the alarm system to alert the user.

If there is a tool tracker attached to the tool, the current axis of the surgical tool 13 can be determined using the tool tracker information. Otherwise, the current axis of the surgical tool 13 can be determined using the encoder values of the motors of the robotic arm 11 knowing the position of the robot tracker on the surgical robotic system.

The estimated end point position of the tool tip can be determined differently depending on whether the surgical tool 13 can move freely in the tool guide or whether there is a mechanical stop. If there is a stop, the information about the maximum depth that the surgical tool can reach is used along the current axis of the surgical tool to estimate its end point position. If there is no stop, it is assumed that the surgical tool 13 can reach the same depth as the planned end point of the planned trajectory and this planned end point is then projected onto the current axis of the surgical tool to determine the estimated end point position of the tool tip.

The maximal allowed angular difference is, advantageously, a value comprised between 0.4 and 1.4°, such as 0.9°, and the maximal allowed distance difference is, advantageously, a value comprised between 0.4 and 1.5mm, such as 0.9mm.

The alarm system may be chosen among various technologies, such as optical system, or an audio system. Advantageously the alarm system comprises LEDs integrated to the robotic arm, or any other part of the surgical robotic system, which change color when there is an alert. For example, those LEDs could be green when there is no issue and become red when the alarm is activated. The color of the LEDs can also change progressively according to the value of the computed difference, for example from green to different shades of yellow/orange when the value of the computed difference approaches the safety criterion and then to red when this criterion is reached. Alternatively, instead of changing color, the LEDs could start flashing at different frequencies depending on the value of the computed difference.

With the proposed system, the load applied to the anatomical structure can be reduced by preventing the surgical robotic system to apply excessive load on the anatomical structure during a surgical intervention by allowing a controlled deviation from the planned trajectory. Indeed, without the invention, it may happen that the surgical robotic system applies a load greater than 100N on the anatomical structure, whereas the invention limits it to the chosen maximal allowed force value Fmax, generally between 25 and 35 N while ensuring a controlled and acceptable deviation from the planned trajectory.

### Operation of the robotic surgical system to drive a screw into a hole previously drilled in a bone

In this embodiment, the planned trajectory is a linear path in a hole previously drilled in an anatomical structure such as the pedicle of vertebra. The robotic arm holds a surgical screwdriver with a screw at its end, the screw forming the tip of the surgical tool. In a preliminary approach, the tip of the screw is brought in contact with an entry point of the drilled hole in the bone positioning the robotic arm according to the reference pose.

Using the localization system, the surgical robotic system is servo-controlled on the patient tracker and compensates in real-time the motion of the bone, which can be due to patient's breathing, any motion created by the screw itself, or any other interaction on the bone. The reference pose is also updated to take into account this servoing.

Once the robotic arm is positioned according to the reference pose, the robotic arm pose information is stored, in the control unit with the other stored data, using the current robotic arm pose, the load applied to the robotic arm is measured using the at least one load measurement means and the control unit uses the measured load to determines the external torque value of each motor. If none of the external torque value is greater than the maximal external torque value allowed for the motor, the control unit then generates, for each motor, an adjusted command using its external torque value, the stored default compensation model and the robotic arm pose information. The robotic arm pose information provides the lever arms value of each motor which are required by the compensation model to determine the compensation term. It is possible that the adjusted command of a motor does not differ from the one of the reference pose since the compensation term is null if the external torque value of the motor is lower than its starting external torque value defined by the compensation model.

Then, the adjusted commands are applied to their respective motor to reduce the load applied to the bone. The user continues to manipulate the surgical tool while the robotic arm is still servo-controlled to track the bone motion in real time and the steps described above are continuously repeated, for example each 5 ms until the tool tip reaches the planned end point position of the planned trajectory. The robotic arm pose information may be updated regularly, each time the steps described above are repeated, with the current robotic arm pose or only stored once as previously described.

During the operation of the robotic surgical system, the model of compensation, used by the control unit, is changed to the second compensation model once a mechanical bond is formed between the bone and the surgical tool and/or screw. Indeed, once this mechanical bond is formed, there is no more risk of deviating from the current surgical tool orientation and so more deviation can be allowed from the planned trajectory using the second compensation model to reduce more the load applied to the anatomical structure. This change of compensation model can be done manually by the user, by pressing a specific button when he feels that the mechanical bond has been formed, or automatically.

When this change is done automatically, the control unit computes a distance between the current position of the screw with the planned end point position of the planned trajectory. The current position of the screw is determined using the length of the screwdriver and the screw, and the position information from the tool tracker.

The compensation model is changed when this distance is lower than a proximity value. This proximity value advantageously corresponds to a percentage of the length of the screw. The proximity value is for example equal to 20% of the length of the screw (so when 80% of the length of the screw is inside the bone) with a minimal allowed value and maximal allowed value respectively equal to 2mm and 8 mm.

In this embodiment an external torque value is used, and the robotic arm pose is stored in the control unit, but of course it would have been possible to use an external wrench value without storing and/or updating the robotic arm pose as previously described. Also, it could have been possible to apply this method without servo-controlling the robotic arm and updating the reference pose.

The skilled person will be able to adjust the stored data to customize the compensation models, and or used a different number of compensation model, for each specific surgical operation.

In the above-described method, it is considered that the adjustment of the command is made for each motor of the robotic arm, which is the preferred embodiment. However, in other embodiments, it would be possible to consider only a subset of the motors of the robotic arm (the subset comprising at least one motor) in the controlled loop implemented by the control unit. For example, if the robotic arm comprises seven motor it would be possible to consider only six of the seven motors.

## Claims

1. System for a manipulation of a surgical tool (13) by a user to treat an anatomical structure according to a planned trajectory, said system comprising:
- a surgical robotic system (1) configured to align the surgical tool (13) according to the planned trajectory, the surgical robotic system (1) comprising:
* a robotic arm (11) comprising a plurality of segments driven by motors, the robotic arm (11) being able to be positioned according to a reference pose configured to align the surgical tool (13) with the planned trajectory;
* at least one load measurement means to measure a mechanical load applied to the robotic arm (11);
* an end-effector (12) mechanically coupled to a distal end (111) of the robotic arm (11) and comprising a tool holder (14) to accommodate the surgical tool (13);
- a control unit (4) coupled to the surgical robotic system (1),
the system being **characterized in that** the control unit is configured to implement at least one controlled loop to reduce a mechanical load applied to the anatomical structure once the robotic arm (11) is positioned according to the reference pose, the controlled loop comprising:
a. a step of measuring the mechanical load applied to the robotic arm (11) using the at least one load measurement means, the mechanical load comprising an inherent mechanical load which is inherent to the robotic arm and an external mechanical load which is applied to the motors of the robotic arm by an external influence;
b. a step of determining an adjusted command for at least one motor, using the following method:
i. determining an external mechanical load value based on the measured load;
ii. determining a compensation term based on the external mechanical load value and on data stored in the control unit (4), said data comprising at least one set of parameters defining a respective compensation model generated by the control unit, each compensation model defining, for each external mechanical load value (L), a corresponding compensation term (Δ) which increases with the external mechanical load value (L);
iii. generating the adjusted command based on the compensation term (Δ) and the reference pose;
c. a step of applying to said at least one motor the respective adjusted command to reduce the mechanical load applied to the anatomical structure.

2. System according to claim 1 wherein the controlled loop comprises, before step ii), a step of storing and/or updating, in the data stored in the control unit, a robotic arm pose information with a current robotic arm pose, this step being done only once during a first iteration of the controlled loop or repeated for each iteration of the controlled loop.

3. System according to any of claim 1 to 2 wherein:
- the at least one load measurement means comprises a plurality of torque measurement sensors configured to measure a torque of each motor; and
- the control unit (4) is configured to determine the external mechanical load based on the external torque value associated to each motor by subtracting from the measured torque value of each motor inherent torque values of the robotic arm (11).

4. System according to any of claim 1 to 2 wherein:
- the at least one load measurement means comprises a force-torque sensor configured to measure a wrench applied to the robotic arm; and
- the control unit (4) is configured to determine the external mechanical load based on the external wrench value by subtracting from the measured wrench value inherent wrench values of the robotic arm (11).

5. System according to any of claim 1 to 4 comprising a localization system (3) coupled to the control unit (4).

6. System according to claim 5, comprising a patient tracker (30), localizable by the localization system (3), rigidly attached to a base structure fixed with respect to the anatomical structure and wherein the surgical robotic system (1) is servo-controlled on the movements of the patient tracker (30).

7. System according to claim 6, wherein the control loop is configured to update the reference pose of the robotic arm (11) at the beginning of each iteration of the controlled loop to take into account the servoing of the surgical robotic system (1) on the movements of the patient tracker (30).

8. System according to any of claim 5 to 7 comprising a robot tracker (31), localizable by the localization system (3), rigidly attached to a part of the surgical robotic system (1).

9. System according to any of claim 1 to 8 comprising an alarm system to inform a user when at least one safety criterion of the robotic arm is not met, the safety criterion being chosen among:
- a maximal allowed angular difference between a current axis of the surgical tool and an axis of the planned trajectory; and
- a maximal allowed distance difference between an estimated end point position of the tool tip and a planned end point position of the planned trajectory;
wherein the control unit (4) is configured to:
- determine the current axis of the surgical tool (13) and the estimated end point position of the tool tip along the current axis of the surgical tool (13);
- compute an angular difference between the current axis of the surgical tool (13) and the axis of the planned trajectory;
- compute a distance difference between the estimated end point position of the tool tip and the planned end point position of the planned trajectory;
- if the angular difference and/or the distance difference is greater than the respective safety criterion, send a signal to trigger the alarm system to alert the user.

10. System according to any one of claims 1 to 9 wherein the stored data comprise at least two sets of parameters defining each a different compensation model and the control unit (4) is configured to select a compensation model among said different compensation models and to determine, for each motor, the compensation term of step b) using the selected compensation model and the external mechanical load value.

11. System according to claim 10 comprising a button configured to, when pressed by a user, change the compensation model used by the control unit (4) in step b).

12. System according to the combination of claim 5 and claim 10 wherein:
- the system comprises a tool tracker, localizable by the localization system (3), rigidly attached to the surgical tool (13);
- the control unit (4) is configured to implement, before step b), a step a') of automatically changing the compensation model used in step b) according to a current position of a tool tip of the surgical tool (13) along the planned trajectory using the following method:
- determining the current position of the tool tip of the surgical tool (13) along the planned trajectory using a position information of the tool tracker from the localization system (3);
- computing a distance between the current position of the tool tip with a planned end point position of the planned trajectory;
- if the distance is lower than a proximity value, changing the compensation model used in step b).

13. System according to any of claims 1 to 12, in combination with claim 3 or claim 4 wherein the stored data comprise a maximal allowed force value (Lmax) applicable to the anatomical structure and the control unit (4) is configured to:
- based on the maximal allowed force value, compute a maximal external torque value for each motor or a maximal external wrench value for the robotic arm;
- send a stop command to the robotic arm to stop movement of the robotic arm if the external torque value applied to a motor or the external wrench value measured in step b) is greater than the respective maximal external torque value or maximal allowed external wrench value.

## Patentansprüche

1. System für eine Manipulation eines chirurgischen Werkzeugs (13) durch einen Benutzer zum Behandeln einer anatomischen Struktur gemäß einer geplanten Trajektorie, wobei das System umfasst:
- ein chirurgisches Robotersystem (1), ausgelegt zum Ausrichten des chirurgischen Werkzeugs (13) gemäß der geplanten Trajektorie, wobei das chirurgische Robotersystem (1) umfasst:
* einen Roboterarm (11), der mehrere von Motoren angetriebene Segmente umfasst, wobei der Roboterarm (11) fähig ist, gemäß einer Referenzstellung positioniert zu werden, die dazu konfiguriert ist, das chirurgische Werkzeug (13) mit der geplanten Trajektorie auszurichten;
* wenigstens eine Lastmesseinrichtung zum Messen einer mechanischen Last, die an dem Roboterarm (11) anliegt;
* einen Endeffektor (12), der mechanisch an ein distales Ende (111) des Roboterarms (11) gekoppelt ist und einen Werkzeughalter (14) zum Aufnehmen des chirurgischen Werkzeugs (13) umfasst;
- eine Steuereinheit (4), die an das chirurgische Robotersystem (1) gekoppelt ist, wobei das System **dadurch gekennzeichnet ist, dass** die Steuereinheit dazu ausgelegt ist, wenigstens eine Steuerschleife zu implementieren, um eine mechanische Last, die auf die anatomische Struktur ausgeübt wird, zu verringern, sobald der Roboterarm (11) gemäß der Referenzstellung positioniert ist, wobei die Steuerschleife umfasst:
a. einen Schritt des Messens der mechanischen Last, die an dem Roboterarm (11) anliegt, unter Verwendung der wenigstens einen Lastmesseinrichtung, wobei die mechanische Last eine inhärente mechanische Last, die dem Roboterarm inhärent ist, und eine externe mechanische Last, die durch eine externe Einwirkung auf die Motoren des Roboterarms ausgeübt wird, umfasst;
b. einen Schritt des Bestimmens eines angepassten Befehls für wenigstens einen Motor unter Verwendung des folgenden Verfahrens:
i. Bestimmen eines Werts der externen mechanischen Last auf der Grundlage der gemessenen Last;
ii. Bestimmen eines Kompensationsterms auf der Grundlage des Werts der externen mechanischen Last und von in der Steuereinheit (4) gespeicherten Daten, wobei die Daten wenigstens einen Satz von Parametern umfassen, die ein entsprechendes Kompensationsmodell definieren, das von der Steuereinheit erzeugt wird, wobei jedes Kompensationsmodell für jeden Wert der externen mechanischen Last (L) einen entsprechenden Kompensationsterm (Δ) definiert, der mit dem Wert der externen mechanischen Last (L) zunimmt;
iii. Erzeugen des angepassten Befehls auf der Grundlage des Kompensationsterms (Δ) und der Referenzstellung;
c. einen Schritt des Anlegens des entsprechenden angepassten Befehls an den wenigstens einen Motor, um die mechanische Last zu verringern, die auf die anatomische Struktur ausgeübt wird.

2. System nach Anspruch 1, wobei die Steuerschleife vor Schritt ii) einen Schritt des Speicherns und/oder Aktualisierens, in den in der Steuereinheit gespeicherten Daten, einer Roboterarmstellungsinformation mit einer aktuellen Roboterarmstellung umfasst, wobei dieser Schritt nur einmal während einer ersten Iteration der Steuerschleife durchgeführt oder für jede Iteration der Steuerschleife wiederholt wird.

3. System nach einem der Ansprüche 1 bis 2, wobei:
- die wenigstens eine Lastmesseinrichtung mehrere Drehmomentmesssensoren umfasst, die dazu ausgelegt sind, ein Drehmoment jedes Motors zu messen; und
- die Steuereinheit (4) dazu ausgelegt ist, die externe mechanische Last auf der Grundlage des Werts des externen Drehmoments, das jedem Motor zugeordnet ist, durch Subtrahieren des Werts des inhärenten Drehmoments jedes Motors von dem gemessenen Drehmomentwert des Roboterarms (11) zu bestimmen.

4. System nach einem der Ansprüche 1 bis 2, wobei:
- die wenigstens eine Lastmesseinrichtung einen Kraft-Drehmoment-Sensor umfasst, der dazu ausgelegt ist, eine auf den Roboterarm ausgeübte Verdrehung zu messen; und
- die Steuereinheit (4) dazu ausgelegt ist, die externe mechanische Last auf der Grundlage des Werts der externen Verdrehung durch Subtrahieren von inhärenten Verdrehungswerten des Roboterarms (11) von dem gemessenen Verdrehungswert zu bestimmen.

5. System nach einem der Ansprüche 1 bis 4 umfassend ein Lokalisierungssystem (3), das an die Steuereinheit (4) gekoppelt ist.

6. System nach Anspruch 5, umfassend einen Patienten-Tracker (30), der von dem Lokalisierungssystem (3) lokalisierbar ist und starr an einer Basisstruktur befestigt ist, die bezogen auf die anatomische Struktur fixiert ist, und wobei das chirurgische Robotersystem (1) auf der Grundlage der Bewegungen des Patienten-Trackers (30) servogesteuert wird.

7. System nach Anspruch 6, wobei die Steuerschleife dazu ausgelegt ist, die Referenzstellung des Roboterarms (11) zu Beginn jeder Iteration der Steuerschleife zu aktualisieren, um die Servobewegung des chirurgischen Robotersystems (1) auf der Grundlage der Bewegungen des Patienten-Trackers (30) zu berücksichtigen.

8. System nach einem der Ansprüche 5 bis 7, umfassend einen Patienten-Tracker (31), der von dem Lokalisierungssystem (3) lokalisierbar ist und starr an einem Teil des chirurgischen Robotersystems (1) befestigt ist.

9. System nach einem der Ansprüche 1 bis 8, umfassend ein Alarmsystem zum Informieren eines Benutzers, wenn wenigstens ein Sicherheitskriterium des Roboterarms nicht erfüllt ist, wobei das Sicherheitskriterium ausgewählt ist aus:
- einer maximal zulässigen Winkeldifferenz zwischen einer aktuellen Achse des chirurgischen Werkzeugs und einer Achse der geplanten Trajektorie; und
- einer maximal zulässigen Abstandsdifferenz zwischen einer geschätzten Endpunktposition der Werkzeugspitze und einer geplanten Endpunktposition der geplanten Trajektorie;
wobei die Steuereinheit (4) ausgelegt ist zum:
- Bestimmen der aktuellen Achse des chirurgischen Werkzeugs (13) und der geschätzten Endpunktposition der Werkzeugspitze entlang der aktuellen Achse des chirurgischen Werkzeugs (13);
- Berechnen einer Winkeldifferenz zwischen der aktuellen Achse des chirurgischen Werkzeugs (13) und der Achse der geplanten Trajektorie;
- Berechnen einer Abstandsdifferenz zwischen der geschätzten Endpunktposition der Werkzeugspitze und der geplanten Endpunktposition der geplanten Trajektorie;
- wenn die Winkeldifferenz und/oder die Abstandsdifferenz größer als das jeweilige Sicherheitskriterium ist, Senden eines Signals zum Auslösen des Alarmsystems zum Warnen des Benutzers.

10. System nach einem der Ansprüche 1 bis 9, wobei die gespeicherten Daten wenigstens zwei Sätze von Parametern umfassen, die jeweils ein anderes Kompensationsmodell definieren, und die Steuereinheit (4) dazu ausgelegt ist, ein Kompensationsmodell unter den verschiedenen Kompensationsmodellen auszuwählen und für jeden Motor den Kompensationsterm von Schritt b) unter Verwendung des ausgewählten Kompensationsmodells und des Werts der externen mechanischen Last zu bestimmen.

11. System nach Anspruch 10, umfassend eine Taste, die dazu ausgelegt ist, bei Betätigung durch einen Benutzer das von der Steuereinheit (4) bei Schritt b) verwendete Kompensationsmodell zu ändern.

12. System nach der Kombination von Anspruch 5 und Anspruch 10, wobei:
- das System einen Werkzeug-Tracker umfasst, der von dem Lokalisierungssystem (3) lokalisierbar ist und starr an dem chirurgischen Werkzeug (13) befestigt ist;
- die Steuereinheit (4) dazu ausgelegt ist, vor Schritt b) einen Schritt a') des automatischen Änderns des in Schritt b) verwendeten Kompensationsmodells gemäß einer aktuellen Position einer Werkzeugspitze des chirurgischen Werkzeugs (13) entlang der geplanten Trajektorie unter Verwendung des folgenden Verfahrens zu implementieren:
- Bestimmen der aktuellen Position der Werkzeugspitze des chirurgischen Werkzeugs (13) entlang der geplanten Trajektorie unter Verwendung einer Positionsinformation des Werkzeug-Trackers von dem Lokalisierungssystem (3);
- Berechnen eines Abstands zwischen der aktuellen Position der Werkzeugspitze und einer geplanten Endpunktposition der geplanten Trajektorie;
- wenn der Abstand kleiner als ein Näherungswert ist, Ändern des bei Schritt b) verwendeten Kompensationsmodells.

13. System nach einem der Ansprüche 1 bis 12 in Kombination mit Anspruch 3 oder Anspruch 4, wobei die gespeicherten Daten einen Wert der maximal zulässigen Kraft (Lmax) umfassen, die auf die anatomische Struktur anwendbar ist, und die Steuereinheit (4) dazu ausgelegt ist:
- auf der Grundlage des Werts der maximal zulässigen Kraft einen maximalen Wert des externen Drehmoments für jeden Motor oder einen maximalen Wert des externen Drehmoments für den Roboterarm zu berechnen;
- Senden eines Stopp-Befehls an den Roboterarm, um die Bewegung des Roboterarms zu stoppen, wenn der Wert des an einen Motor angelegten externen Drehmoments oder der bei Schritt b) gemessene Wert des externen Drehmoments größer als der entsprechende maximale Wert des externen Drehmoments oder der maximal zulässige Wert des externen Drehmoments ist.

## Revendications

1. Système de manipulation d'un outil chirurgical (13) par un utilisateur pour traiter une structure anatomique selon une trajectoire planifiée, ledit système comprenant :
- un système robotique chirurgical (1) configuré pour aligner l'outil chirurgical (13) selon la trajectoire planifiée, le système robotique chirurgical (1) comprenant :
* un bras robotique (11) comprenant plusieurs segments entraînés par des moteurs, le bras robotique (11) pouvant être positionné selon une pose de référence configurée pour aligner l'outil chirurgical (13) sur la trajectoire planifiée ;
* au moins un moyen de mesure de charge pour mesurer une charge mécanique appliquée au bras robotique (11) ;
* un effecteur terminal (12) accouplé mécaniquement à une extrémité distale (111) du bras robotique (11) et comportant un support d'outil (14) pour recevoir l'outil chirurgical (13) ;
- une unité de commande (4) couplée au système robotique chirurgical (1),
le système étant **caractérisé en ce que** l'unité de commande est configurée pour mettre en œuvre au moins une boucle commandée pour réduire une charge mécanique appliquée à la structure anatomique une fois que le bras robotique (11) est positionné selon la pose de référence, la boucle commandée comprenant :
a. une étape de mesure de la charge mécanique appliquée au bras robotique (11) à l'aide de l'au moins un moyen de mesure de charge, la charge mécanique comprenant une charge mécanique inhérente qui est inhérente au bras robotique et une charge mécanique externe qui est appliquée aux moteurs du bras robotique par une influence externe ;
b. une étape de détermination d'une instruction ajustée pour au moins un moteur, en utilisant le procédé suivant :
i. détermination d'une valeur de charge mécanique externe en fonction de la charge mesurée ;
ii. détermination d'un terme de compensation en fonction de la valeur de charge mécanique externe et de données mémorisées dans l'unité de commande (4), lesdites données comprenant au moins un ensemble de paramètres définissant un modèle de compensation respectif généré par l'unité de commande, chaque modèle de compensation définissant, pour chaque valeur de charge mécanique externe (L), un terme de compensation (Δ) correspondant qui augmente avec la valeur de charge mécanique externe (L) ;
iii. génération de l'instruction ajustée en fonction du terme de compensation (Δ) et de la pose de référence ;
c. une étape d'application, audit au moins un moteur, de l'instruction ajustée respective pour réduire la charge mécanique appliquée à la structure anatomique.

2. Système selon la revendication 1 dans lequel la boucle commandée comprend, avant l'étape ii), une étape de mémorisation et/ou de mise à jour, dans les données mémorisées dans l'unité de commande, d'informations de pose de bras robotique avec une pose de bras robotique actuelle, cette étape n'étant effectuée qu'une fois au cours d'une première itération de la boucle commandée ou répétée pour chaque itération de la boucle commandée.

3. Système selon l'une quelconque des revendications 1 à 2 dans lequel :
- l'au moins un moyen de mesure de charge comprend une pluralité de capteurs de mesure de couple configurée pour mesurer un couple de chaque moteur ; et
- l'unité de commande (4) est configurée pour déterminer la charge mécanique externe en fonction de la valeur de couple externe associée à chaque moteur en soustrayant, de la valeur de couple mesurée de chaque moteur, des valeurs de couple inhérentes du bras robotique (11).

4. Système selon l'une quelconque des revendications 1 à 2 dans lequel :
- l'au moins un moyen de mesure de charge comprend un capteur de force-couple configuré pour mesurer un effort mécanique appliqué au bras robotique ; et
- l'unité de commande (4) est configurée pour déterminer la charge mécanique externe en fonction de la valeur d'effort mécanique externe en soustrayant, de la valeur d'effort mécanique mesurée, des valeurs d'effort mécanique inhérentes du bras robotique (11).

5. Système selon l'une quelconque des revendications 1 à 4 comprenant un système de localisation (3) couplé à l'unité de commande (4).

6. Système selon la revendication 5, comprenant un dispositif de suivi de patient (30), localisable par le système de localisation (3), fixé rigidement à une structure de base fixée par rapport à la structure anatomique et dans lequel le système robotique chirurgical (1) est asservi aux mouvements du dispositif de suivi de patient (30).

7. Système selon la revendication 6, dans lequel la boucle de commande est configurée pour mettre à jour la pose de référence du bras robotique (11) au début de chaque itération de la boucle commandée pour prendre en compte l'asservissement du système robotique chirurgical (1) aux mouvements du dispositif de suivi de patient (30).

8. Système selon l'une quelconque des revendications 5 à 7 comprenant un dispositif de suivi de robot (31), localisable par le système de localisation (3), fixé rigidement à une partie du système robotique chirurgical (1).

9. Système selon l'une quelconque des revendications 1 à 8 comprenant un système d'alarme pour informer un utilisateur lorsqu'au moins un critère de sécurité du bras robotique n'est pas satisfait, le critère de sécurité étant choisi entre :
- une différence angulaire autorisée maximale entre un axe courant de l'outil chirurgical et un axe de la trajectoire planifiée ; et
- une différence de distance autorisée maximale entre une position estimée de point d'extrémité du bout de l'outil et une position planifiée de point d'extrémité de la trajectoire planifiée ;
dans lequel l'unité de commande (4) est configurée pour :
- déterminer l'axe courant de l'outil chirurgical (13) et la position estimée de point d'extrémité du bout de l'outil le long de l'axe courant de l'outil chirurgical (13) ;
- calculer une différence angulaire entre l'axe courant de l'outil chirurgical (13) et l'axe de la trajectoire planifiée ;
- calculer une différence de distance entre la position estimée de point d'extrémité du bout de l'outil et la position planifiée du point d'extrémité de la trajectoire planifiée ;
- si la différence angulaire et/ou la différence de distance sont supérieures au critère de sécurité respectif, envoyer un signal de déclenchement du système d'alarme pour avertir l'utilisateur.

10. Système selon l'une quelconque des revendications 1 à 9 dans lequel les données mémorisées comprennent au moins deux ensembles de paramètres définissant chacun un modèle de compensation différent et l'unité de commande (4) est configurée pour sélectionner un modèle de compensation entre lesdits modèles de compensation différents et pour déterminer, pour chaque moteur, le terme de compensation de l'étape b) en utilisant le modèle de compensation choisi et la valeur de charge mécanique externe.

11. Système selon la revendication 10 comprenant un bouton configuré pour, lorsqu'il est appuyé par un utilisateur, changer le modèle de compensation utilisé par l'unité de commande (4) à l'étape b).

12. Système selon la combinaison de la revendication 5 et de la revendication 10 dans lequel :
- le système comprend un dispositif de suivi d'outil, localisable par le système de localisation (3), fixé rigidement à l'outil chirurgical (13) ;
- l'unité de commande (4) est configurée pour mettre en œuvre, avant l'étape b), une étape a') de modification automatique du modèle de compensation utilisé à l'étape b) selon une position courante d'un bout d'outil de l'outil chirurgical (13) le long de la trajectoire planifiée selon le procédé suivant :
- détermination de la position courante du bout d'outil de l'outil chirurgical (13) le long de la trajectoire planifiée en utilisant des informations de position du dispositif de suivi d'outil depuis le système de localisation (3) ;
- calcul d'une distance entre la position courante du bout d'outil et une position planifiée de point d'extrémité de la trajectoire planifiée ;
- si la distance est inférieure à une valeur de proximité, modification du modèle de compensation utilisé à l'étape b).

13. Système selon l'une quelconque des revendications 1 à 12, en combinaison avec la revendication 3 ou la revendication 4, dans lequel les données mémorisées comprennent une valeur de force autorisée maximale (Lmax) applicable à la structure anatomique et l'unité de commande (4) est configurée pour :
- en fonction de la valeur de force autorisée maximale, calculer une valeur de couple externe maximale pour chaque moteur ou une valeur d'effort mécanique externe maximale pour le bras robotique ;
- envoyer une instruction d'arrêt au bras robotique pour arrêter le mouvement du bras robotique si la valeur de couple externe appliquée à un moteur ou la valeur d'effort mécanique externe mesurée à l'étape b) est supérieure à la valeur de couple externe maximale ou à la valeur d'effort mécanique externe autorisée maximale respective.
